# FASCICULE DE BREVET EUROPEEN

(11) **EP 3 854 355 B1**
(45) Date de publication et mention de la délivrance du brevet: **31.05.2023**
(21) Numéro de dépôt: 21150837.9
(22) Date de dépôt: 11.01.2021
(51) Int. Cl.: A61F 2/42

(54) **COMPOSANT IMPLANTABLE A MOYEN D'ANCRAGE AMELIORE POUR PROTHESE DE CHEVILLE ET PROTHESE DE CHEVILLE COMPRENANT UN TEL COMPOSANT**
IMPLANTIERBARE KOMPONENTE MIT VERBESSERTER VERANKERUNG FÜR KNÖCHELPROTHESE UND EINE SOLCHE KOMPONENTE UMFASSENDE KNÖCHELPROTHESE
IMPLANTABLE COMPONENT WITH IMPROVED ANCHORING MEANS FOR ANKLE PROSTHESIS AND ANKLE PROSTHESIS COMPRISING SUCH A COMPONENT

(30) Priorité: 22.01.2020 FR 2000621
(43) Date de publication de la demande: 28.07.2021
(73) Titulaire: In2Bones, 69130 Ecully (FR)
(72) Inventeur: LEEMRIJSE, Thibaut Jean Pierre Henry, 1200 Bruxelles (BE); PUTZEYS, Pit, L8119 Bridel (LU); PAUL, Laurent François René, 1495 Mellery (BE); AGREN, Per-Henrik, 11140 Stockholm (SE); BESSE, Jean-Luc Pierre Marie, 69970 Chaponnay (FR)
(74) Mandataire: Weber, Jean-François

(56) Documents cités:
- WO-A1-2019/045412
- WO-A1-2019/220048
- US-A1- 2003 204 265
- US-A1- 2011 035 019

## Description

L'invention se rapporte au domaine général des prothèses de cheville, c'est-à-dire des dispositifs implantables destinés au remplacement d'articulations de chevilles, en particulier dans le cadre d'un traitement orthopédique.

L'invention concerne plus précisément un composant implantable de prothèse de cheville, ledit composant comprenant un corps principal pourvu d'une face de contact osseux qui est prévue pour être agencée en contact avec une zone d'un corps osseux d'une cheville et qui s'étend selon un plan de contact médian, ledit composant comprenant au moins un moyen d'ancrage qui fait saillie de ladite face de contact osseux pour ancrer ledit composant dans ledit corps osseux.

L'invention concerne également une prothèse de cheville comprenant au moins un composant implantable.

Afin de traiter certaines pathologies osseuses de la cheville, telle que l'arthrose, entraînant une dégradation ou une disparition du cartilage articulaire, il est connu de procéder à une arthrodèse de l'articulation tibio-tarsienne. Une telle opération d'arthrodèse vise à limiter, voire bloquer totalement, la mobilité de cheville par ostéosynthèse, afin de faire cesser la douleur articulaire ressentie par le patient. Si l'arthrodèse de l'articulation tibio-tarsienne donne généralement satisfaction, son principal inconvénient réside précisément dans la suppression de la mobilité de l'articulation, qui doit alors être compensée autant que de possible par les autres articulations de la jambe du patient. Une longue période d'adaptation est alors nécessaire au patient pour retrouver, après opération, des capacités de locomotion satisfaisantes. En outre, le blocage de l'articulation tibio-tarsienne engendre des contraintes mécaniques élevées sur les articulations voisines, lesquelles sont alors exposées à un risque élevé de dégénérescence précoce.

C'est la raison pour laquelle il a été proposé de procéder, dans certains cas de figures, à une arthroplastie de la cheville comme alternative à l'arthrodèse, c'est-à-dire au remplacement total ou partiel de l'articulation endommagée de la cheville par une articulation prothétique, artificielle.

Il a ainsi été introduit diverses prothèses de cheville formée d'un ou plusieurs composants implantables, en vue de remplacer en tout ou partie d'une articulation endommagée de la cheville. On connaît ainsi en particulier, une prothèse de cheville formée d'une pluralité de composants implantables, à savoir un composant talaire et un composant tibial, respectivement destinés à être ancrés au talus et au tibia, et un patin en plastique qui est, quant à lui, prévu pour être intercalé entre le composant talaire et le composant tibial et venir s'articuler en contact avec le composant talaire.

Le recours à de telles prothèses connues permet, contrairement à l'arthrodèse classique, de conserver une bonne mobilité de la cheville du patient, facilitant ainsi la marche, et de préserver les différentes articulations du pied et de la jambe du patient. Il a cependant été observé que les prothèses de cheville connues restent encore perfectibles, en particulier en matière de tenue mécanique de l'ancrage osseux de leur(s) composant(s), mais également en matière de praticité et de précision de mise en place du ou des composants implantables dans le corps du patient.

Le document WO 2019/045412 A1 décrit un composant implantable de prothèse de cheville selon le préambule de la revendication 1.

Les objets assignés à la présente invention visent en conséquence à proposer un nouveau composant implantable de prothèse de cheville, ainsi qu'une nouvelle prothèse de cheville comprenant un tel composant, ce dernier comprenant un moyen d'ancrage du composant dans un corps osseux d'une cheville permettant à la fois un ancrage osseux particulièrement fiable du composant et une mise en place particulièrement aisée et précise dudit composant dans le corps du patient.

Un autre objet de l'invention vise à proposer un nouveau composant implantable et une nouvelle prothèse de cheville dont la mise en place est à la fois rapide et particulièrement peu traumatisante pour le patient.

Un autre objet de l'invention vise à proposer un nouveau composant implantable et une nouvelle prothèse de cheville, qui sont particulièrement robustes et résistants.

Un autre objet de l'invention vise à proposer un nouveau composant implantable et une nouvelle prothèse de cheville de conception simple et dont la fabrication est particulièrement aisée.

Un autre objet de l'invention vise à proposer un nouveau composant implantable et une nouvelle prothèse de cheville permettant de réduire le coût de l'intervention de traitement chirurgical d'une cheville.

Un autre objet de l'invention vise à proposer un nouveau composant implantable et une nouvelle prothèse de cheville permettant de réduire le risque pour la santé du patient.

Un autre objet de l'invention vise à proposer un nouveau composant implantable et une nouvelle prothèse de cheville permettant de traiter une pathologie osseuse du patient de façon particulièrement efficace et rapide.

Les objets assignés à l'invention sont atteints à l'aide d'un composant implantable de prothèse de cheville selon la revendication 1.

Les objets assignés à l'invention sont également atteints à l'aide d'une prothèse de cheville, comprenant au moins un composant implantable, selon la revendication 14.

D'autres particularités et avantages de l'invention apparaîtront et ressortiront plus en détails à la lecture de la description faite ci-après, en référence aux dessins annexés, donnés uniquement à titre d'exemples illustratifs et non limitatifs, dans lesquels :
Figure 1 illustre, selon une vue en perspective, un mode de réalisation préférentiel d'un composant implantable conforme à l'invention, lequel constitue avantageusement un composant tibial de prothèse de cheville ;
Figure 2 illustre, selon une vue médiale, le composant implantable de la figure 1 ;
Figure 3 illustre, selon une vue postérieure, le composant implantable des figures 1 et 2 ;
Figure 4 illustre, selon une vue antérieure, le composant implantable des figures 1 à 3 ;
Figure 5 illustre, selon une vue de dessus, le composant implantable des figures 1 à 4 ;
Figure 6 illustre, selon une vue de dessous, le composant implantable des figures 1 à 5 ;
Figure 7 illustre, selon une vue en perspective, un mode de réalisation d'une prothèse de cheville conforme à l'invention, laquelle prothèse comprend le composant implantable des figures 1 à 6 ;
Figure 8 illustre, selon une vue en perspective, la prothèse de cheville de la figure 7, implantée au niveau d'un pied gauche d'un patient en remplacement de l'articulation tibio-tarsienne anatomique.

Selon un premier aspect, l'invention concerne, d'une part, un composant 1 implantable de prothèse de cheville, prévu pour être rapporté à un corps osseux Ti, Ta d'une cheville à traiter. Le composant 1 implantable conforme à l'invention comprend à ce titre un corps principal 2 pourvu d'une face 3 de contact osseux, qui est prévue pour être agencée en contact avec une zone d'un corps osseux Ti, Ta d'une cheville, ainsi qu'au moins un moyen d'ancrage 4 qui fait saillie de ladite face 3 de contact osseux pour ancrer ledit composant 1 dans ledit corps osseux Ti, Ta. Selon un deuxième aspect, l'invention concerne une prothèse 5 de cheville comprenant au moins un tel composant 1 implantable.

La prothèse 5 de cheville comprenant un composant 1 implantable conforme à l'invention constitue un dispositif médical, implantable par voie chirurgicale dans le corps d'un patient, qui est destiné au remplacement de tout ou partie d'une articulation tibio-tarsienne dudit patient. A ce titre, une telle prothèse 5 et son composant 1 implantable sont conçus pour être insérés et intercalés entre une extrémité distale d'un tibia Ti et un talus Ta (ou astragale) correspondant de la cheville du patient. Avantageusement, le tibia Ti et / ou le talus Ta considérés auront fait l'objet, préalablement à la mise en place de la prothèse 5 dans le corps du patient, d'une préparation adéquate, et par exemple d'une extraction d'éléments cartilagineux et de coupes osseuses, de sorte à supprimer tout ou partie des surfaces articulaires naturelles de l'articulation tibio-tarsienne à traiter.

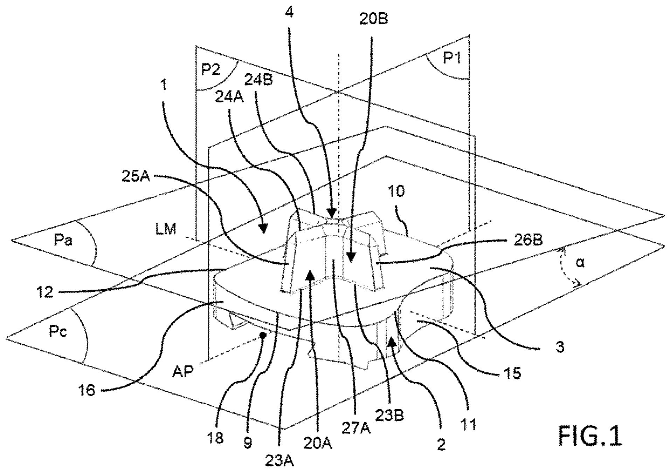

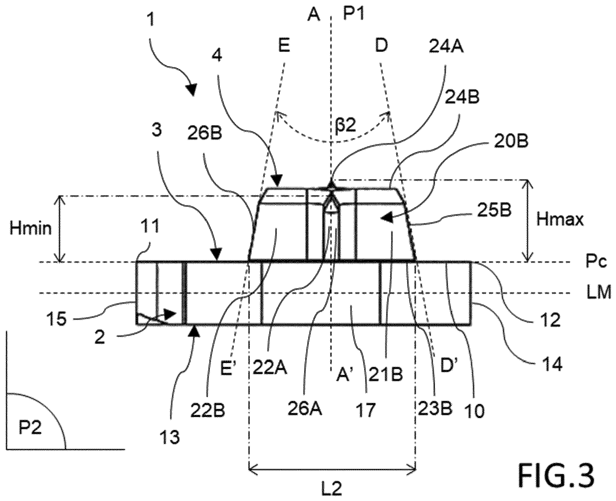

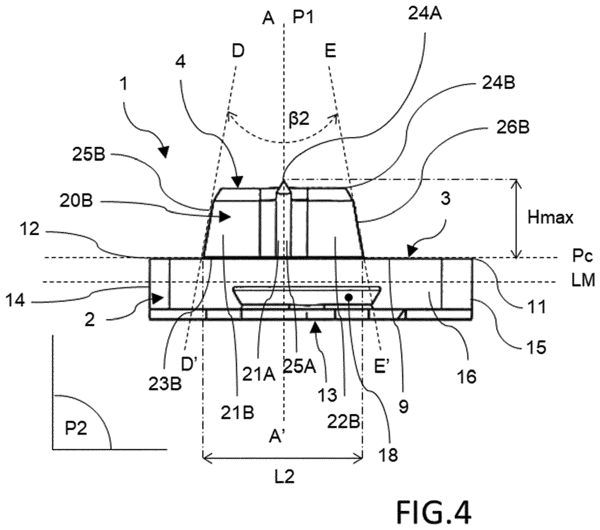

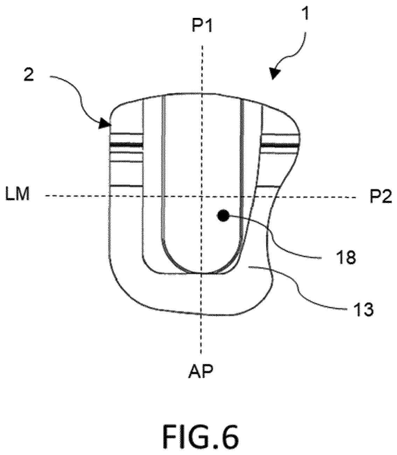

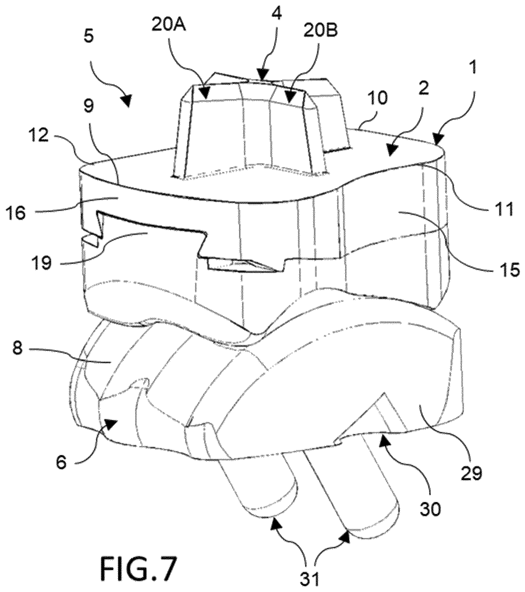

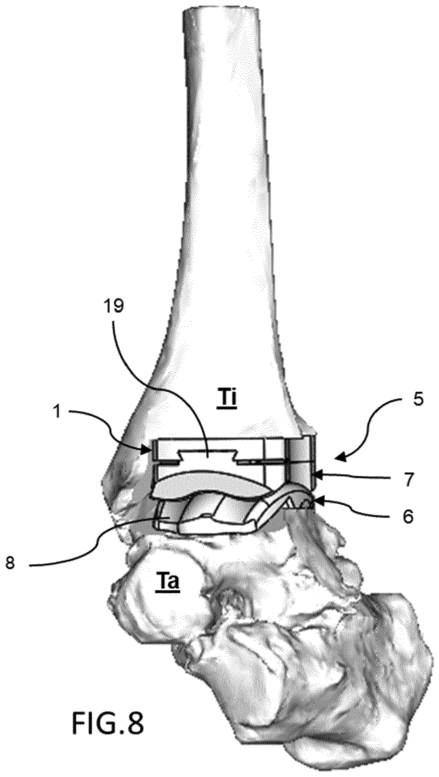

De manière avantageuse, le composant 1 implantable de l'invention constitue plus spécifiquement un composant tibial 1 de prothèse 5 de cheville, dont la face 3 de contact osseux est donc à ce titre spécifiquement prévue pour être agencée en contact avec une zone d'une extrémité distale d'un tibia Ti. Un mode de réalisation préférentiel d'un composant 1 implantable, conforme à l'invention, constituant un tel composant tibial 1 de prothèse 5 de cheville est illustré en exemple aux figures 1 à 6 notamment. Réciproquement, la prothèse 5 de cheville de l'invention comprend avantageusement un tel composant 1 implantable constituant un composant 1 tibial et dont la face 3 de contact osseux est prévue pour être agencée en contact avec une zone d'une extrémité distale d'un tibia Ti. Un mode de réalisation d'une telle prothèse 5 de cheville est illustré en exemple à la figure 7. Le composant 1 implantable et la prothèse 5 illustrée en exemples aux figures sont destinés à être mise en place au niveau d'un pied gauche d'un patient, comme illustré en situation à la figure 8. Bien évidemment, l'invention recouvre également un composant 1 et une prothèse 5 qui seraient destinés à être mis en place au niveau d'un pied droit du patient. Avantageusement, ceux-ci seraient alors définis par symétrie, par rapport au plan sagittal du patient, des composant 1 et prothèse 5 illustrés aux figures.

Avantageusement, comme dans le mode de réalisation illustré aux figures, ladite prothèse 5 est conçue pour remplacer totalement l'articulation tibio-tarsienne concernée (prothèse totale de cheville, PTC). A ce titre, la prothèse 5 de comprend au moins un composant 1 implantable, qui est conforme à l'invention et qui constitue un composant 1 tibial, et un composant talaire 6 qui est lui aussi destiné à être implanté dans le corps d'un patient dont on souhaite traiter l'articulation d'une cheville. Tandis que le composant tibial 1 est prévu pour être agencé, rapporté, en contact avec une zone d'une extrémité distale d'un tibia Ti, conformément à ce qui précède, le composant talaire 6 est prévu pour être agencé, rapporté, en contact avec une zone d'un talus Ta (ou astragale) correspondant d'une cheville du patient. Lesdits composant tibial 1 et composant talaire 6 sont alors conçus pour coopérer, directement ou indirectement, de manière à former une articulation prothétique apte à reproduire le plus fidèlement possible la cinématique naturelle de la cheville anatomique.

Pour autant, le composant implantable conforme à l'invention pourrait éventuellement constituer un composant talaire d'une prothèse de cheville. Il pourrait par ailleurs constituer un composant d'une prothèse conçue pour remplacer uniquement une partie d'une articulation tibio-tarsienne à traiter. Ainsi, ledit composant implantable selon l'invention pourrait constituer un composant tibial conçu pour coopérer, directement ou indirectement avec une surface articulaire anatomique d'un talus Ta, dans l'hypothèse où seule l'extrémité distale du tibia Ti est abimée et nécessite d'être traitée et équipée d'une surface articulaire prothétique. Alternativement ledit composant implantable pourrait constituer un composant talaire de prothèse de cheville conçu pour coopérer, directement ou indirectement avec une surface articulaire anatomique d'un tibia Ti, dans l'hypothèse où seule la surface articulaire du talus Ta est abimée et nécessite d'être traitée et équipée d'une surface articulaire prothétique.

Réciproquement, la prothèse 2 de cheville de l'invention pourrait comprendre un composant 1 implantable conforme à l'invention, unique ou non, qui constituerait un composant talaire de ladite prothèse 2, ou encore comprendre un composant tibial et un composant talaire qui sont tous deux conformes à l'invention.

Dans le cas où la prothèse 5 est conçue pour remplacer totalement une articulation tibio-tarsienne, ladite prothèse 5 de cheville peut en outre avantageusement comprendre, outre lesdits composants tibial 1 et talaire 6, un composant intermédiaire 7 (ou patin, ou encore insert) qui est conçu pour être intercalé entre ledit composant talaire 6 et ledit composant tibial 1, comme illustré en exemples aux figures 7 à 10. Ledit composant intermédiaire 7 comprend avantageusement une surface articulaire intermédiaire conçue pour coopérer avec une surface articulaire talaire 8 correspondante du composant talaire 6. Ledit composant intermédiaire 7 comprend une surface d'interface tibiale avec le composant tibial 1, opposée à ladite surface articulaire intermédiaire. Dans les modes de réalisation des composant 1 et prothèse 5 illustrés aux figures, un tel composant intermédiaire 7 est conçu pour être solidarisé au composant tibial 1, et immobilisé par rapport à ce dernier, par l'intermédiaire de ladite surface d'interface tibiale, de sorte à former une prothèse 5 de cheville dite « *bi-composants ».* Toutefois, selon une variante non illustrée, le composant tibial et le composant intermédiaire pourraient être alternativement conçus de sorte à autoriser une mobilité du composant intermédiaire par rapport au composant tibial, par coopération de la surface d'interface tibiale du composant intermédiaire avec une surface correspondante du composant tibial. La prothèse de cheville formerait alors une prothèse dite « *à trois composants ».* On notera que, pour former une prothèse 5 de cheville dite *« bi-composants »,* il est également possible de prévoir que les composants tibial 1 et intermédiaire 7 susvisés ne soient pas distincts l'un de l'autre, mais forment au contraire un seul et même composant, éventuellement monolithique. Néanmoins, le composant intermédiaire 7 constituant une pièce d'usure, du fait de son interaction avec la surface articulaire talaire 8 du composant talaire 6, il est avantageux que le composant intermédiaire 7 puisse être distinct du composant tibial 1, et solidarisé à ce dernier de manière amovible de sorte à en permettre le remplacement ultérieur si nécessaire. Dans le cas où le composant intermédiaire 7 est distinct du composant tibial 1, et solidarisé ou non à ce dernier, ledit composant intermédiaire 7 est préférentiellement en tant que tel, une pièce monobloc en un matériau présentant un faible coefficient de frottement, par exemple un matériau plastique tel qu'un polyéthylène haute densité (PEHD). Il peut s'agir, par exemple, d'une pièce usinée ou moulée.

La face 3 de contact osseux du corps principal 2 du composant 1 implantable de l'invention s'étend selon un (unique) plan Pc de contact médian, avantageusement d'une part entre un bord antérieur 9 et un bord postérieur 10 opposé selon une direction d'extension antéro-postérieure AP du corps principal 2 et d'autre part, entre un bord latéral 11 et un bord médial 12 opposé selon une direction d'extension latéro-médiale LM du corps principal 2. De préférence, ladite face 3 de contact osseux est sensiblement plane, comme dans le mode de réalisation illustré aux figures. Ladite face 3 de contact osseux est alors avantageusement prévue pour être agencée en contact direct avec ladite zone du corps osseux Ti, Ta, laquelle zone aura été préalablement préparée pour présenter une surface correspondante sensiblement plane. Le corps principal 2 du composant 1 sera donc avantageusement prévu pour être positionné en contact direct plan sur plan avec ladite zone du corps osseux Ti, Ta. Pour autant, la face 3 de contact osseux pourrait alternativement ne pas être strictement plane, bien que s'étendant toutefois selon ledit plan Pc de contact médian.

De préférence, le corps principal 2 du composant 1 implantable constitue une pièce monolithique, formée en un matériau avantageusement biocompatible et résistant à l'usure. Avantageusement, ledit corps principal 2 est en un matériau métallique, par exemple en titane, en alliage chrome-cobalt CrCo, ou encore en acier inoxydable. Le corps principal 2 du composant 1 implantable est avantageusement une pièce de fonderie et / ou usinée dans la masse. Bien évidemment, d'autres matériaux convenables pourront être envisagés, tel que par exemple un matériau céramique, un matériau polymère (PEEK, etc.) ou encore un matériau composite, ainsi que d'autres modes de fabrication (par injection, moulage, frittage, etc.), pour autant que le ou les matériaux retenus confère au corps principal 2 une tenue mécanique suffisante pour l'application visée.

Dans le mode de réalisation préférentiel illustré aux figures, où le composant 1 constitue plus spécifiquement un composant tibial 1, le corps principal 2 comprend également une autre face 13 (ou face inférieure), opposée à ladite face 3 de contact osseux, destinée à venir en contact avec un composant intermédiaire 7 distinct, tel qu'évoqué ci-avant. Avantageusement, la face inférieure 13 du corps principal 2 s'étend selon un plan médian qui est sensiblement parallèle audit plan Pc de contact médian, ledit composant tibial 1 se présentant ainsi sous la forme générale d'une plaque, d'une épaisseur typiquement comprise entre 5 et 10 mm. Tel qu'illustré, le corps principal 2 du composant tibial 1 peut également comporter une face médiale tibiale 14 et une face latérale tibiale 15 opposée, lesquelles relient respectivement le bord médial 12 et le bord latéral 11 de la face 3 de contact osseux à la face inférieure 13 du corps principal 2. Avantageusement, ledit bord médial 12 du corps principal 2 est sensiblement rectiligne et ladite face médiale tibiale 14 est sensiblement plane, de sorte à ce que le chirurgien puisse positionner précisément ledit composant tibial 1 dans le corps du patient, en alignant ladite face médiale tibiale 14 le long d'une coupe rectiligne réalisée au niveau de la malléole interne de la cheville du patient. Cela permet de réduire avantageusement les zones de coupes osseuses non recouvertes, qui pourraient favoriser l'apparition de géodes ou de kystes. En outre, le corps principal 2 du composant tibial 1 peut comporter une face antérieure tibiale 16 et une face postérieure tibiale 17 opposée, lesquelles relient quant à elles respectivement le bord antérieur 9 et le bord postérieur 10 de la face 3 de contact osseux à la face inférieure 13 du corps principal 2.

Il est ici à noter que les termes « *postérieur », « antérieur », « médial »* et « *latéral* » sont préférentiellement utilisés dans la présente description pour qualifier des éléments ou des caractéristiques du composant 1 et de la prothèse 5 en lien avec leur orientation respective relativement au corps du patient, en usage normal desdits composant 1 et prothèse 5. Ainsi, le terme « *médial »* est préférentiellement utilisé pour désigner un élément du composant 1 et / ou de la prothèse 5 qui destiné à être positionné et orienté du côté le plus proche de l'axe medio-sagittal (ou axe médian) du corps du patient, autrement dit le côté orienté vers l'intérieur du pied et de la jambe du patient. Par opposition, le terme « *latéral »* est utilisé en relation avec le côté le plus éloigné de l'axe medio-sagittal. Suivant la même logique, les termes « *postérieur »* et *« antérieur »* renvoient de préférence respectivement à un positionnement vers l'arrière, respectivement vers l'avant, relativement au plan frontal du patient.

Toujours dans le mode de réalisation préférentiel illustré aux figures, le composant tibial 1 est conçu pour être solidarisé audit composant intermédiaire 7 distinct, de sorte à supprimer avantageusement tout degré de liberté entre le composant tibial 1 et le composant intermédiaire 7. Une telle solidarisation de ces derniers permet d'améliorer avantageusement la stabilité de l'articulation prothétique formée par la prothèse 5. A ce titre, le composant tibial 1 comprend avantageusement un premier élément 18 de solidarisation qui est complémentaire d'un deuxième élément 19 de solidarisation porté par le composant intermédiaire 7. Par exemple, lesdits premier et deuxième éléments 18, 19 de solidarisation complémentaires sont conçus pour permettre un assemblage en queue d'aronde du composant intermédiaire 7 au composant tibial 1. Ainsi, la face inférieure 13 du composant tibial 1 peut être avantageusement pourvue d'une rainure 18, par exemple de section trapézoïdale, qui forme avantageusement ledit premier élément 18 de solidarisation (ou organe femelle en queue d'aronde). Le composant intermédiaire 7 pourra alors être réciproquement pourvu d'un tenon 19, formant ledit deuxième élément 19 de solidarisation (ou organe mâle en queue d'aronde), et de forme et dimensions complémentaires de celle de ladite rainure 18. Bien évidemment, une configuration inversée pourrait tout à fait être envisagée, ladite face inférieure 13 étant pourvue dudit tenon, ledit composant intermédiaire 7 étant réciproquement pourvue de ladite rainure. D'autres moyens de solidarisation convenables pourraient également être envisagés.

Comme déjà évoqué précédemment, le composant 1 implantable de l'invention comprend au moins un moyen d'ancrage 4 qui se projette à partir de la face 3 de contact osseux du corps principal 2 du composant 1, pour permettre un ancrage de ce dernier dans le corps osseux Ti, Ta concerné, c'est-à-dire dans la masse osseuse d'une extrémité distale d'un tibia Ti ou d'un talus Ta. Ledit moyen d'ancrage 4 est avantageusement destiné à venir se loger, lors de la mise en place du composant 1 implantable dans le corps du patient, dans au moins un logement correspondant pratiqué au préalable et / ou lors de la mise en place du composant 1 implantable par le chirurgien dans la masse osseuse du corps osseux Ti, Ta concerné. Selon l'invention, ledit moyen d'ancrage 4 comprend deux paires 20A, 20B d'ailes 21A, 22A, 21B, 22B d'ancrage, qui s'étendent chacune respectivement dans un plan P1, P2 orthogonal audit plan Pc de contact médian de la face 3 de contact osseux entre une première extrémité 23A, 23B reliée à ladite face 3 de contact osseux et une deuxième extrémité 24A, 24B (libre) opposée, lesdits plans P1, P2 d'extension respectifs des paires 20A, 20B d'ailes 21A, 22A, 21B, 22B d'ancrage étant sécants. Les premier et deuxième plans P1, P2 d'extension des première et deuxième paires 20A, 20B d'ailes 21A, 22A, 21B, 22B sont donc sécants selon une droite A-A' perpendiculaire au plan Pc de contact médian de la face 3 de contact osseux du corps principal 2 du composant 1 implantable.

Le moyen d'ancrage 4 du composant 1 implantable comprend ainsi :
- un première paire 20A d'ailes 21A, 22A d'ancrage, qui sont coplanaires dans un premier plan P1 d'extension orthogonal audit plan Pc de contact médian de la face 3 de contact osseux, et
- une deuxième paire 20B d'ailes 21B, 22B d'ancrage, qui s'étendent de manière coplanaires dans un deuxième plan P2 d'extension, lequel est, d'une part, orthogonal audit plan Pc de contact médian de la face 3 de contact osseux et, d'autre part, sécant audit premier plan P1 d'extension de la première paire 20 d'ailes 21A, 22A d'ancrage.

On entend ici de préférence par « *aile »*, un élément qui s'étend majoritairement dans un plan selon des dimensions supérieures à une épaisseur mesurée dans une direction perpendiculaire audit plan et qui est donc à ce titre avantageusement sensiblement plat, bidimensionnel.

Le caractère orthogonal des plans P1, P2 d'extension des paires 20A, 20B d'ailes 21A, 22A, 21B, 22B d'ancrage permet avantageusement de simplifier la préparation par le chirurgien de la zone du corps osseux Ti, Ta au contact de laquelle le corps principal 2 du composant 1 implantable est destiné à être rapporté audit corps osseux Ti, Ta. Il permet également un positionnement et une insertion particulièrement simples et précis, avantageusement par impaction du composant 1 implantable, du moyen d'ancrage 4 dans la masse osseuse du corps osseux Ti, Ta, tout en favorisant une mise en contact intime de la face 3 de contact osseux du corps principal 2 du composant 1 implantable avec ladite zone du corps osseux Ti, Ta. Le caractère sécant des plans P1, P2 d'extension des paires 20A, 20B d'ailes 21A, 22A, 21B, 22B d'ancrage permet avantageusement de solidariser de manière particulièrement fiable et efficace le composant 1 implantable au corps osseux Ti, Ta, en interdisant en particulier tout mouvement de translation ou de rotation du corps principal 2 du composant 1 implantable relativement audit corps osseux Ti, Ta.

Conformément à l'invention, les deuxièmes extrémités 24A, 24B des paires 20A, 20B d'ailes 21A, 22A, 21B, 22B d'ancrage sont par ailleurs inscrites dans un plan Pa d'attaque qui est incliné par rapport au plan Pc de contact médian de la face 3 de contact osseux du composant 1, ce qui permet de faciliter grandement l'insertion et la mise en place du composant 1 implantable dans le corps du patient, entre l'extrémité distale du tibia Ti et le talus Ta correspondant d'un pied du patient, et ce tout en conservant une distraction articulaire limitée.

La mise en oeuvre d'un tel moyen d'ancrage 4 comprenant deux paires 20A, 20B d'ailes 21A, 22A, 21B, 22B d'ancrage, qui s'étendent chacune respectivement dans un plan P1, P2 orthogonal audit plan Pc de contact médian de la face 3 de contact osseux entre une première extrémité 23A, 23B reliée à ladite face 3 de contact osseux et une deuxième extrémité 24A, 24B (libre) opposée, lesdits plans P1, P2 d'extension respectifs des paires 20A, 20B d'ailes 21A, 22A, 21B, 22B d'ancrage étant sécants, lesdites deuxièmes extrémités 24A, 24B des paires 20A, 20B d'ailes 21A, 22A, 21B, 22B d'ancrage étant inscrites dans un plan Pa d'attaque incliné par rapport audit plan Pc de contact médian, est tout particulièrement avantageuse dans le cas particulier où le composant 1 implantable constitue un composant tibial 1 de prothèse de cheville, dans la mesure notamment où l'extrémité distale du tibia Ti est en pratique bien plus difficile d'accès pour le chirurgien que le talus Ta correspondant, tant pour la préparation de la zone destinée à recevoir le composant tibial 1 que pour la mise en place et l'ancrage ultérieurs de ce dernier dans la masse osseuse du tibia Ti. Pour autant, la mise en oeuvre d'un tel moyen d'ancrage 4 peut également s'avérer intéressante dans le cas où composant 1 implantable de l'invention constitue un composant talaire 6 de prothèse de cheville, dans la mesure où l'accès physique au talus Ta, bien que plus facile, n'en demeure pas moins relativement complexe et limité en pratique. Ainsi, l'opération de mise en place du composant 1 implantable de l'invention, et plus généralement de la prothèse 5 comprenant ce dernier, peut être réalisée de manière plus aisée, plus précise et plus rapide par le chirurgien.

La limitation de la distraction articulaire nécessaire pour la mise en place du composant 1 implantable, et plus généralement de la prothèse 5 de cheville, est en outre particulièrement bénéfique pour le patient, puisqu'elle permet de réduire le risque de traumatisme tissulaire associé à l'opération de traitement de la cheville du patient, et donc de favoriser un rétablissement rapide de ce dernier.

Selon une variante préférentielle, retenue dans le mode de réalisation illustrée aux figures, le plan d'attaque Pa est incliné par rapport au plan Pc de contact médian selon une pente déclinante du bord antérieur 9 en direction du bord postérieur 10 de la face 3 de contact osseux. Une telle variante est particulièrement bien adaptée à une insertion et une mise en place du composant 1 implantable dans le corps du patient par une voie d'abord antérieure (c'est-à-dire par l'avant du pied du patient). Alternativement, le plan d'attaque Pa pourrait, à l'inverse, être incliné par rapport au plan Pc de contact médian selon une pente déclinante du bord postérieur 10 en direction du bord antérieur 9 de la face 3 de contact osseux, en vue d'une insertion et d'une mise en du composant 1 implantable dans le corps du patient par une voie d'abord postérieure (c'est-à-dire par l'arrière du pied du patient). Alternativement encore, le plan d'attaque Pa pourrait être incliné par rapport au plan Pc de contact médian selon une pente déclinante du bord médial 12 en direction du bord latéral 11 de la face 3 de contact osseux, en vue d'une insertion et d'une mise du composant 1 implantable dans le corps du patient par une voie d'abord médiale (c'est-à-dire par le côté intérieur du pied du patient). Cependant, compte tenu de la configuration de la cheville anatomique humaine, des implantations par voie d'abord postérieure ou médiale s'avèrent généralement plus complexes et plus risquées à mettre en oeuvre pour le chirurgien et pour le patient, de la sorte que la voie d'abord antérieure est généralement préférée.

Quelle que soit la voie d'abord préférée, et donc le sens de la pente du plan Pa d'attaque retenu, le compromis entre la fiabilité de l'ancrage du composant 2 dans la masse osseuse du corps osseux Ti, Ta et la praticité d'insertion et de mise en place du composant 1 implantable dans le corps du patient peut être avantageusement amélioré lorsque le plan Pa d'attaque est incliné par rapport au plan Pc de contact médian selon un angle α compris entre 3° et 30°, de préférence compris entre 3° et 15°, et de préférence encore compris entre 5° et 8°, et par exemple égal à 6,3° environ.

Avantageusement, les premières extrémités 23A, 23B des paires 20A, 20B d'ailes 21A, 22A, 21B, 22B d'ancrage sont reliées directement en contact avec la face 3 de contact osseux, de sorte que lesdites des paires 20A, 20B d'ailes 21A, 22A, 21B, 22B d'ancrage s'étendent à partir de cette dernière jusqu'à leur deuxième extrémité 24A, 24B respective. Plus avantageusement encore, lesdites paires 20A, 20B d'ailes 21A, 22A, 21B, 22B sont reliées directement en contact avec la face 3 de contact osseux, au niveau de leurs premières extrémités 23A, 23B respectives, par une arête. La jonction entre lesdites paires 20A, 20B d'ailes 21A, 22A, 21B, 22B d'ancrage et la surface de la face 3 de contact osseux du corps principal 2 est donc avantageusement dépourvue de congé, ou de zone de transition arrondie, ce qui permet notamment de faciliter une mise en contact précise et intime de la face 3 de contact osseux avec la zone correspondante du corps osseux Ti, Ta lors de la mise en place du composant 1 dans le corps du patient. Alternativement, la face 3 de contact osseux pourrait être pourvue d'une cuvette à partir du fond de laquelle s'étendraient les paires 20A, 20B d'ailes 21A, 22A, 21B, 22B d'ancrage avec un congé dont la hauteur serait inférieure à la profondeur de la cuvette, de sorte que le congé ne s'étendrait pas au-delà de la surface de la face 3 de contact osseux.

Avantageusement, comme dans le mode de réalisation préférentiel illustré aux figures, les ailes 21A, 22A, 21B, 22B de chaque paires 20A, 20B d'ailes 21A, 22A, 21B, 22B d'ancrage se prolongent l'une l'autre à partir de la droite A-A' d'intersection des plans P1, P2 d'extension respectifs desdites paires 20A, 20B d'ailes 21A, 22A, 21B, 22B d'ancrage. Chaque paire 20A, 20B d'ailes 21A, 22A, 21B, 22B s'étend donc de manière sensiblement continue, ce qui permet notamment de simplifier la conception et la fabrication du moyen d'ancrage 4 et du composant 1 implantable.

Afin notamment de simplifier la conception du composant 1 et d'équilibrer au mieux l'ancrage du composant 1 selon les directions respectives des plans P1, P2 d'extension des deux paires 20A, 20B d'ailes 21A, 22A, 21B, 22B, les ailes 21A, 22A, 21B, 22B de chacune desdites paires 20A, 20B d'ailes 21A, 22A, 21B, 22B d'ancrage sont préférentiellement conformées de manière que leurs projections orthogonales dans le plan Pc de contact médian sont symétriques entre elles par rapport au plan P1, P2 d'extension de l'autre desdites paires 20A, 20B d'ailes 21A, 22A, 21B, 22B d'ancrage. Ainsi, comme illustrés aux figures, les ailes 21A, 22A de la première paire 20A d'ailes21A, 22A sont conformées de manière que leurs projections orthogonales respectives dans le plan Pc de contact médian sont avantageusement symétriques l'une de l'autre par rapport au plan P2 d'extension de la deuxième paire 20B d'ailes 21B, 22B et, réciproquement, les ailes 21B, 22B de la deuxième paire 20B d'ailes 21B, 22B sont conformées de manière que leurs projections orthogonales respectives dans le plan Pc de contact médian sont avantageusement symétriques l'une de l'autre par rapport au plan P1 d'extension de la première paire 20A d'ailes 21A, 22A.

Comme illustré aux figures, chacune desdites ailes 21A, 22A, 21B, 22B d'ancrage est avantageusement pourvue d'un bord extérieur latéral 25A, 26A, 25B, 26B qui relie entre elles les première et deuxième extrémités 23A, 23B, 24A, 24B de paires 20A, 20B d'ailes 21A, 22A, 21B, 22B d'ancrage. Lesdits bords extérieurs latéraux 25A, 26A, 25B, 26B des ailes 21A, 22A, 21B, 22B d'une même paire 20A, 20B d'ailes 21A, 22A, 21B, 22B s'étendent préférentiellement selon des directions B-B', C-C', D-D', E-E' médianes qui sont obliques et convergentes en s'éloignant du plan Pc de contact médian. Les bords extérieurs latéraux 25A, 26A, 25B, 26B s'étendent donc de manière inclinée, oblique, par rapport plan Pc de contact médian, selon des directions B-B', C-C', D-D', E-E' médianes qui sont par ailleurs sécantes deux à deux en des points d'intersection situés au-delà du plan Pc de contact médian et du plan Pa d'attaque, de sorte que les bords extérieurs latéraux 25A, 26A, 25B, 26B des ailes 21A, 22A, 21B, 22B d'une même paire 20A, 20B d'ailes 21A, 22A, 21B, 22B ne sont donc pas parallèles l'un à l'autre. Les bords externes latéraux 25A, 26A, des ailes 21A, 22A de la première paire 20A d'ailes 21A, 22A d'ancrage s'étendent ainsi avantageusement respectivement selon des directions B-B', C-C' médianes qui forment entres elles un premier angle β1, tandis que les bords externes latéraux 25B, 26B des ailes 21B, 22B de la deuxième paire 20B d'ailes 21B, 22B d'ancrage s'étendent avantageusement respectivement selon des directions D-D', E-E' médianes qui forment entres elle un deuxième angle β2. Pour simplifier la conception du moyen d'ancrage 4, ledit premier et deuxième angles β1, β2 sont préférentiellement choisis de valeur identiques. Une telle configuration des bords externes latéraux 25A, 26A, 25B, 26B des ailes 21A, 22A, 21B, 22B contribue avantageusement à une insertion facile, en particulier par impaction, du moyen d'ancrage 4 dans la masse osseuse du corps osseux Ti, Ta considéré.

De manière plus préférentielle encore, les ailes 21A, 22A, 21B, 22B de chacune desdites paires 20A, 20B d'ailes 21A, 22A, 21B, 22B sont conformées de manière que leurs projections orthogonales dans le plan Pc de contact médian sont symétriques entre elles par rapport au plan d'extension P1, P2 de l'autre desdites paires 20A, 20B d'ailes 21A, 22A, 21B, 22B, et les bords extérieurs latéraux 25A, 26A, 25B, 26B des ailes 21A, 22A, 21B, 22B d'une même paire 20A, 20B d'ailes 21A, 22A, 21B, 22B s'étendent selon des directions B-B', C-C', D-D', E-E' médiane obliques et convergentes, comme envisagé ci-dessus. Ainsi, les bords extérieurs latéraux 25A, 26A, 25B, 26B des ailes 21A, 22A, 21B, 22B de chacune des paires 20A, 20B d'ailes 21A, 22A, 21B, 22B sont inscrites dans un cône fictif de révolution qui s'étend en hauteur selon une droite confondue avec la droite A-A' d'intersection des plans P1, P2 d'extension des paires 20A, 20B d'ailes 21A, 22A, 21B, 22B. Ainsi, le moyen d'ancrage 4 peut être positionné dans la masse osseuse, en particulier par impaction, de manière particulièrement facile et précise selon une trajectoire d'implantation bien maîtrisée. Qui plus est, une telle conformation du moyen d'ancrage 4 est particulièrement bien adaptée dans le cas particulier où le composant 1 implantable constitue un composant tibial 1, compte tenu de la forme globalement conique de l'extrémité distale du tibia Ti (figure 8).

Avantageusement, lesdites directions B-B', C-C', D-D', E-E' médianes d'extension des bords extérieurs latéraux 25A,26A,25B,26B des ailes 21A, 22A, 21B, 22B d'une même paire 20A, 20B d'ailes 21A, 22A, 21B, 22B sont sécantes selon un angle β1, β2 compris entre 3° et 75°, et de préférence compris entre 10° et 30°. De manière plus préférentielle encore, ledit angle β1, β2 est sensiblement égal à 20°, de manière notamment à autoriser la mise en oeuvre de grandes largeurs d'ailes 21A, 22A, 21B, 22B, et donc à optimiser la tenue de l'ancrage, tout en limitant le risque d'interaction desdites ailes 21A, 22A, 21B, 22B avec l'os cortical du tibia Ti, plus dur que la matière osseuse formant la partie centrale du tibia Ti et donc susceptible de s'opposer à la parfaite insertion du moyen d'ancrage 4, dans le cas où le composant 1 implantable constitue un composant tibial 1. Une telle valeur d'angle β1, β2 peut également s'avérer intéressante en matière de tenue mécanique du moyen d'ancrage 4 dans la masse osseuse, dans le cas où le composant 1 implantable constitue un composant talaire de prothèse de cheville, bien que l'absence de partie corticale au niveau du talus Ta puisse éventuellement autoriser une valeur d'angle β1, β2 supérieure à 20°.

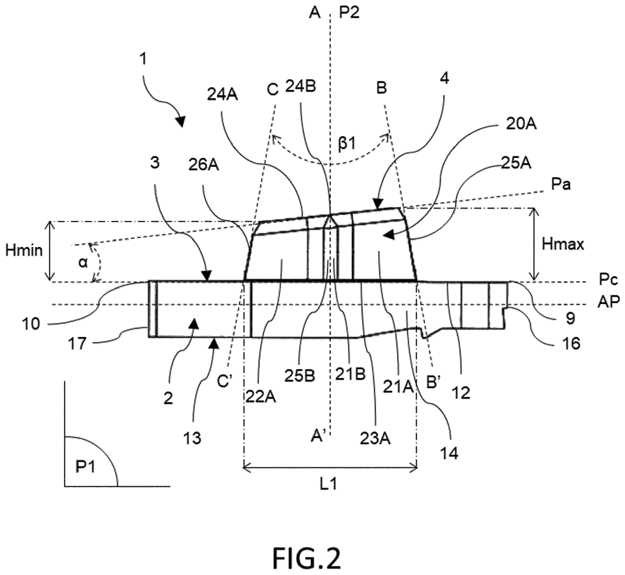

De manière avantageuse, les ailes 21A, 22A, 21B, 22B du moyen d'ancrage 4 du composant 1 implantable s'étendent entre leurs première et deuxième extrémités 23A, 23B, 24A, 24B selon une hauteur Hmin, Hmax au moins égale à 3 mm et au plus égale à 30 mm, en particulier dans le cas où composant 1 implantable constitue un composant tibial 1 (comme dans le mode de réalisation préférentiel illustré aux figures). Comme illustré aux figures 2 à 4, ladite hauteur des ailes 21A, 22A, 21B, 22B est avantageusement mesurée à partir de la surface de la face 3 de contact osseux du corps principal 2, perpendiculairement au plan Pc de contact médian selon lequel s'étend ladite la face 3 de contact osseux. Compte tenu de l'inclinaison, par rapport au plan Pc de contact médian, du plan Pa d'attaque dans lequel les deuxièmes extrémités 24A, 24B respectives des paires 20A, 20B d'ailes 21A, 22A, 21B, 22B d'ancrage sont inscrites (et indépendamment de la valeur de l'angle α d'inclinaison dudit plan Pa d'attaque), le (ou les) point desdites deuxièmes extrémités 24A, 24B le plus proche de la surface de la face 3 de contact osseux est donc positionné à une distance Hmin d'au moins 3 mm de cette dernière. Réciproquement, le (ou les) point desdites deuxièmes extrémités 24A, 24B le plus éloigné de la surface de la face 3 de contact osseux est donc positionné à une distance Hmax d'au plus 30 mm de cette dernière. Une hauteur ainsi optimisée des ailes 21A, 22A, 21B, 22B du moyen d'ancrage 4 contribue avantageusement à l'obtention d'un excellent compromis entre la robustesse et la fiabilité de l'ancrage du corps principal 2 du composant 1 implantable dans la masse osseuse du corps osseux Ti, Ta et la limitation du risque d'endommagement de ce dernier par le moyen d'ancrage 4 d'une part, et la facilité de mise en place dudit composant 1 dans le corps du patient (tant en matière de préparation préalable de la zone osseuse qu'en matière d'implantation du composant 1 implantable lui-même) d'autre part.

En pratique, ladite hauteur Hmin, Hmax pourra être choisie selon le contexte d'implantation. Par exemple, dans le cas d'une première intervention d'implantation pour une cheville donnée, la hauteur minimale Hmin pourra être avantageusement choisie au moins égale à 3 mm, et la hauteur maximale Hmax pourra être avantageusement choisie au plus égale à 15 mm. Pour une intervention en reprise, c'est-à-dire visant à remplacer un implant existant au niveau d'une cheville donnée, la hauteur minimale Hmin pourra être avantageusement choisie au moins égale à 15 mm, et la hauteur maximale Hmax pourra être avantageusement choisie au plus égale à 30 mm.

Les ailes 21A, 22A, 21B, 22B du moyen d'ancrage 4 présentent chacune de préférence une épaisseur e1, e2 moyenne comprise entre 1 mm et 3 mm, de préférence égale à 1,5 mm environ. Une telle épaisseur e1, e2 permet notamment de conférer aux ailes 21A, 22A, 21B, 22B une rigidité suffisante, d'une part pour éviter une torsion des ailes 21A, 22A, 21B, 22B lors de la mise en place du composant 1 implantable, en particulier lorsque cette dernière est réalisée par impaction, et d'autre part pour garantir un excellent ancrage du composant 1 implantable dans la masse osseuse et une grande robustesse intrinsèque du moyen d'ancrage 4. En outre, le fait que les ailes 21A, 22A, 21B, 22B restent pour autant relativement fines permet de limiter le volume de masse osseuse à enlever lors de la préparation de la zone osseuse concernée et / ou à remplacer par le moyen d'ancrage 4, ce qui simplifie l'opération de préparation du corps osseux Ti, Ta et favorise la tenue du moyen d'ancrage 4, tout en respectant au mieux l'intégrité anatomique.

De préférence, le moyen d'ancrage 4 est agencé sensiblement au centre de la face 3 de contact osseux du corps principal 2 du composant 1 implantable. Une fois implanté dans la masse osseuse du corps osseux Ti, Ta concerné, le composant 1 présentera ainsi une tenue mécanique encore améliorée, le risque de rotation du composant 1 selon une direction orthogonale au plan Pc de contact médian de la face 3 de contact osseux de son corps principal 2 étant en particulier ainsi limité. Dans le cas où ledit composant 1 implantable constitue un composant tibial 1 de prothèse 5 de cheville, il est ainsi avantageusement possible pour le chirurgien, lors de mise de place du composant 1 dans le corps du patient, d'aligner la droite A-A' d'intersection des plans P1, P2 d'extension des paires 20A, 20B d'ailes 21A, 22A, 21B, 22B avec l'axe d'extension moyen respectif du tibia Ti du patient, de manière à assurer une bonne répartition des efforts de compression exercés sur le composant 1 implantable.

De manière avantageuse, le moyen d'ancrage 4 forme, en tant que tel, une pièce monolithique, c'est-à-dire que les deux paires 20A, 20B d'ailes 21A, 22A, 21B, 22B viennent de matière l'une avec l'autre, ce qui permet de simplifier la conception et la fabrication du moyen d'ancrage 4. Alternativement, chacune des ailes 21A, 22A, 21B, 22B ou chacune des paires 20A, 20B d'ailes 21A, 22A, 21B, 22B pourrait être distincte des autres et être assemblées à ces dernières par soudage ou par emboîtement, par exemple. De manière plus avantageuse encore, le moyen d'ancrage 4 forme une pièce monolithique avec le corps principal 2 du composant 1 implantable. Il y a donc continuité de matière entre le corps principal 2 et le moyen d'ancrage 4, ces derniers pouvant dès lors être fabriqués simultanément, de manière simple, rapide et à moindre coût. Avantageusement, le corps principal 2 le moyen d'ancrage 4 pourront alors être tous deux en un matériau métallique, par exemple en titane, en alliage chrome-cobalt CrCo, ou encore en acier inoxydable. Il reste envisageable que le moyen d'ancrage 4, monolithique ou non, puisse être distinct du corps principal 2, et être conçu pour être solidarisé à ce dernier (par exemple par vissage ou encliquetage), avant ou pendant la mise en place du composant 1 implantable dans le corps du patient. Pour autant, une telle conception est moins avantageuse, tant en termes de fiabilité et de robustesse de la liaison entre le corps principal 2 et le moyen d'ancrage 4, qu'en termes de praticité de mise en oeuvre du composant 1 implantable pour le chirurgien. En outre, le recours à un assemblage d'une pluralité de pièces distinctes tend à augmenter la complexité, et donc le coût, de conception et de fabrication.

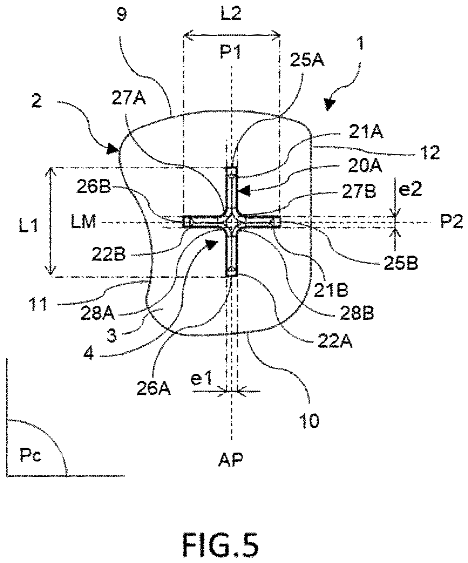

Que le moyen d'ancrage 4 forme ou non, en tant que tel, une pièce monolithique, il est avantageux de prévoir que les paires 20A, 20B d'ailes 21A, 22A, 21B, 22B d'ancrage dudit moyen d'ancrage 4 sont reliées entre elles au niveau de l'intersection de leur plan P1, P2 d'extension respectif par des congés 27A, 28A, 27B, 28B, et non par une arête simple rectiligne (figure 5). De tels congés 27A, 28A, 27B, 28B peuvent alors présenter un rayon de courbure préférentiellement compris entre 2 mm et 15 mm. On renforce ainsi la résistance mécanique intrinsèque du moyen d'ancrage 4, et on limite par ailleurs le risque de formation d'espaces vides au voisinage de l'intersection des paires 20A, 20B d'ailes 21A, 22A, 21B, 22B, une fois le moyen d'ancrage 4 introduit dans la masse osseuse du corps osseux Ti, Ta considéré.

Selon une variante préférentielle, retenue dans le mode de réalisation illustré aux figures, les plans P1, P2 d'extension des première et deuxième paires 20A, 20B d'ailes 21A, 22A, 21B, 22B sont perpendiculaires l'un à l'autre. Le moyen d'ancrage 4 présente ainsi avantageusement une forme en « + *»*, vu en coupe dans un plan parallèle au plan Pc de contact médian. Une telle conception des paires 20A, 20B d'ailes 21A, 22A, 21B, 22B est particulièrement simple à mettre en oeuvre et permet un excellent ancrage du moyen d'ancrage 4 dans la masse osseuse. Par ailleurs, lorsque lesdites paires 20A, 20B d'ailes 21A, 22A, 21B, 22B sont reliées directement à la face 3 de contact osseux par leurs premières extrémités 23A, 23B, une telle conception permet également avantageusement de conférer aux paires 20A, 20B d'ailes 21A, 22A, 21B, 22B une fonction de rigidification mécanique du corps principal 2. Une telle fonction additionnelle peut s'avérer particulièrement intéressante lorsque le corps principal 2 prend la forme d'une plaque, comme envisagé ci-avant et illustré en exemple aux figures, laquelle pourrait être sujette à un risque de torsion, de flexion (en particulier, de flexion médio-latérale), sous les efforts mécaniques exercés sur le composant 1 implantable et la prothèse 5 en utilisation. De préférence, lesdites paires 20A, 20B d'ailes 21A, 22A, 21B, 22B sont respectivement agencés de manière sensiblement parallèle auxdites directions moyennes d'extension antéro-postérieure AP et latéro-médiale LM du corps principal 2 du composant 1 implantable, de sorte à définir respectivement une paire 20A d'ailes 21A, 22A antéro-postérieures et une paire 20B d'ailes 21B, 22B latéro-médiales. Cela permet notamment de simplifier encore la conception et la fabrication du composant 1 implantable et, dans une certaine mesure, de faciliter encore la mise en place dans le corps du patient par le chirurgien, ladite paire 20A d'ailes 21A, 22A antéro-postérieures pouvant jouer un rôle de repère visuel.

De manière plus préférentielle encore, ladite paire 20A d'ailes 21A, 22A antéro-postérieures présente au niveau de sa première extrémité 23A, et suivant ladite direction moyenne d'extension antéro-postérieure AP, une largeur L1 supérieure à une largeur L2 que présente respectivement de ladite paire 20B d'ailes 21B, 22B latéro-médiales au niveau de sa première extrémité 23B respective et suivant ladite direction moyenne d'extension latéro-médiale LM (figures 2 à 5). Avantageusement, la paire 20A d'ailes 21A, 22A antéro-postérieures présente ainsi, au niveau de sa première extrémité 23A et suivant ladite direction moyenne d'extension antéro-postérieure AP, une largeur L1 comprise entre 10 mm et 30 mm, et de préférence égale à 18 mm environ. Réciproquement, la paire 20B d'ailes 21B, 22B latéro-médiales présente, au niveau de sa première extrémité 23B et suivant ladite direction moyenne d'extension latéro-médiale LM, une largeur L2 comprise entre 10 mm et 30 mm, et de préférence égale à 16 mm environ. Cela permet avantageusement d'optimiser l'ancrage du composant 1 implantable dans la masse osseuse, tout en limitant le risque de fragilisation de cette dernière, dans la mesure où tant l'extrémité distale d'un tibia Ti anatomique qu'un talus Ta anatomique présentent généralement une dimension antéro-postérieure supérieure à une dimension latéro-médiale. Pour autant, selon une variante alternative non illustrée et moins avantageuse, les plans P1, P2 d'extension des première et deuxième paires 20A, 20B d'ailes 21A, 22A, 21B, 22B pourraient ne pas être perpendiculaires l'un à l'autre. Les plans P1, P2 d'extension définiraient alors entre eux une paire d'angles obtus et une paire d'angles aigus, et le moyen d'ancrage 4 présenterait donc une forme en « X ».

Avantageusement, les deuxièmes extrémités 24A, 24B libres des paires 20A, 20B d'ailes 21A, 22A, 21B, 22B d'ancrage délimitent des bords d'attaque biseautés, comme illustré en exemple aux figures, de manière à faciliter la pénétration et l'insertion du moyen d'ancrage 4 dans la masse osseuse, en particulier par impaction, tout en limitant le risque d'endommagement de ladite masse osseuse. Avantageusement, les bords extérieurs latéraux 25A, 26A, 25B, 26B de chacune desdites paires 20A, 20B d'ailes 21A, 22A, 21B, 22B d'ancrage sont sensiblement plats, c'est-à-dire qu'ils s'étendent respectivement dans un plan orthogonal au plan P1, P2 d'extension de ladite paire 20A, 20B d'ailes 21A, 22A, 21B, 22. Cela contribue encore à simplifier la conception et la fabrication du moyen d'ancrage 4 et du composant 1 implantable. En outre, de tels bords extérieurs latéraux 25A, 26A, 25B, 26B plats permettent avantageusement de limiter, dans une certaine mesure, le risque de translation du composant 1 implant selon une direction des plans P1, P2 d'extension lors de l'insertion du moyen d'ancrage 1 dans la masse osseuse, et ultérieurement en usage du composant 1 implantable et de la prothèse 5. Alternativement, lesdits bords extérieurs latéraux 25A, 26A, 25B, 26B pourraient être chanfreinés, ou encore biseautés pour faciliter l'insertion du moyen d'ancrage 4 dans la masse osseuse lors de la mise en place du composant 1 implantable dans le corps du patient.

De manière avantageuse, ledit composant 1 implantable est dépourvu de tout moyen, autre que ledit moyen d'ancrage 4, prévu pour assurer ancrer le corps principal 2 du composant 1 implantable dans la masse osseuse du corps osseux Ti, Ta. En particulier, le composant 1 implantable ne comprend avantageusement ni plot ni quille d'ancrage complémentaire audit moyen d'ancrage 4 comprenant lesdites paires 20A, 20B d'ailes 21A, 22A, 21B, 22B d'ancrage. Ledit moyen d'ancrage 4 assure ainsi sensiblement à lui seul la fixation du corps principal 2 du composant 1 dans la masse osseuse du corps osseux Ti, Ta. La conception et la fabrication du composant 1 implantable, et donc de la prothèse 5 de cheville auquel il appartient, s'en trouvent ainsi simplifiées. Par ailleurs, la mise en place du composant 1 implantable dans le corps du patient est alors plus simple, plus rapide et moins douloureuse et moins traumatisante pour le patient, notamment du fait que la préparation de la zone osseuse destinée à recevoir le composant 1 s'en trouve simplifiée puisqu'il n'est pas nécessaire en particulier de prévoir une pluralité de pré-perçage de la masse osseuse pour recevoir une pluralité de moyens d'ancrage distincts. Ceci étant, il reste bien évidemment possible de prévoir au contraire que le composant 1 implantable comprenne, outre ledit moyen d'ancrage 4 comprenant lesdites de paires 20A, 20B d'ailes 21A, 22A, 21B, 22B, des moyens d'ancrage complémentaires distincts, comme par exemple un ou plusieurs plot(s) ou quille(s), faisant eux aussi saillie de la face 3 de contact osseux du corps principal 2 du composant 1 implantable, de préférence alors selon une direction d'extension orthogonale au plan Pc de contact médian de la face 3 de contact osseux.

Optionnellement, les ailes 21A, 22A, 21B, 22B du moyen d'ancrage 4 peuvent être ajourées, c'est-à-dire être pourvues d'un ou plusieurs orifices ou lumières les traversant de part en part, de sorte à favoriser encore l'ancrage du composant 1 implantable dans la masse osseuse par croissance de l'os à l'intérieur desdits orifices ou lumières et la formation de ponts osseux de part et d'autre des ailes 21A, 22A, 21B, 22B. Il sera alors néanmoins très délicat d'extraire ultérieurement ledit composant A hors du corps du patient, sans risque de dégradation de la masse osseuse environnant le moyen d'ancrage 4 de ce dernier. De préférence, comme illustré en exemple aux figures, les ailes 21A, 22A, 21B, 22B du moyen d'ancrage 4 sont au contraire pleines, c'est-à-dire non ajourées, ce qui permet donc de faciliter une extraction future éventuelle du composant 1 implantable hors du corps du patient, par exemple en vue de remplacer ledit composant 1 par un autre composant implantable en cas d'usure, de détérioration à l'usage.

Optionnellement, tout ou partie de la face 3 de contact osseux du corps principal 2 du composant 1 implantable et / ou de la surface des ailes 21A, 22A, 21B, 22B du moyen d'ancrage 4 du composant 1 implantable pourra être pourvue d'un revêtement de surface particulier (par exemple en titane poreux ou en hydroxyapatite), ou avoir fait l'objet d'un traitement mécanique particulier (sablage, rainurage, etc.), afin de favoriser l'accroche osseuse du corps principal 2 et / ou du moyen d'ancrage 4 du composant 1 implantable au corps osseux Ti, Ta auquel ledit composant 1 est destiné à être solidarisé.

Dans le mode de réalisation de la prothèse 5 de cheville illustré à la figure 7, seul le composant tibial 1 est un composant 1 implantable conforme à l'invention. Dans ce mode de réalisation, le composant talaire 6 de la prothèse 5 comprend un corps principal talaire 29 pourvu d'une face 30 talaire de contact osseux prévue pour être agencée en contact avec une zone d'un talus Ta d'une cheville et opposée à la surface articulaire talaire 8 évoquée précédemment. Ladite face 30 talaire de contact osseux s'étend dans un plan (unique) de contact médian. Le composant talaire 6 comprend par ailleurs un moyen d'ancrage talaire 31 formé d'une paire de plots 31 d'ancrage, de forme cylindrique à section circulaire, pour assurer un ancrage du composant talaire 6 dans la masse osseuse du talus Ta. Avantageusement, chacun desdits plots 31 d'ancrage s'étend longitudinalement à partir de la face 30 talaire de contact osseux selon une direction oblique par rapport à cette dernière.

Selon des modes de réalisation alternatifs (non illustrés), la face 30 talaire de contact osseux pourrait ne pas s'étendre dans un plan unique de contact médian, mais par exemple dans deux plans de contact médians sécants. Le moyen d'ancrage talaire 31 pourrait quant à lui alternativement être formé d'un unique plot d'ancrage, par exemple de forme cylindrique à section circulaire, ou encore être formé d'une quille d'ancrage de forme cylindrique ou tronconique. Avantageusement, un tel plot d'ancrage unique ou une telle quille d'ancrage s'étendrait alors longitudinalement à partir de la face talaire de contact osseux selon une direction oblique par rapport à cette dernière.

La mise en oeuvre du composant 1 implantable et de la prothèse 5 de l'invention, dans leurs modes de réalisation illustrés aux figures peut, par exemple et en substance, se dérouler comme suit :
- On pratique une incision dans la peau et les tissus mous de la cheville à traiter du patient, de préférence au niveau de la face avant de la cheville de manière à donner accès à l'articulation tibio-talaire selon une voie d'abord antérieure ;
- On pratique une coupe osseuse plane et horizontale (par rapport à la position verticale naturelle du patient en station debout) au niveau de l'extrémité distale du tibia Ti, de préférence à l'aide d'un guide de coupe adapté, de sorte à former une zone osseuse sensiblement plane destinée à venir en contact avec la face 3 de contact osseux du composant tibial 1 ;
- On pratique une ou plusieurs coupes osseuses dans le talus Ta, de sorte à former une zone osseuse plane ou comportant une pluralité de portions planes sécantes, et destinée à venir en contact avec la face 30 talaire de contact osseux du composant talaire 6 ;
- On pratique, dans ladite zone osseuse préparée dans le talus Ta, un ou plusieurs pré-perçages talaires, destinés à recevoir le moyen d'ancrage talaire31 du composant talaire 6 ;
- On pratique, dans ladite zone osseuse préparée dans le tibia Ti, un pré-perçage tibial destiné à recevoir le moyen d'ancrage 4 du composant tibial 1, à l'aide d'un guide de perçage tibial pourvu d'un orifice traversant dont le pourtour est de forme complémentaire de la section transverse du moyen d'ancrage 4 et d'un moyen de pré-perçage par impaction pourvu d'une portion de travail de forme sensiblement identique à celle dudit moyen d'ancrage 4. Après avoir fixé temporairement le guide de perçage tibial en contact avec la zone osseuse plane du tibia Ti, on réalise le pré-perçage tibial en introduisant à coulissement la portion de travail du moyen de pré-perçage dans l'orifice traversant correspondant du guide de perçage tibial, puis en insérant à force par impaction ladite portion de travail dans la masse osseuse du tibia Ti ;
- Après avoir retiré le guide de perçage tibial, on positionne et maintient le composant tibial 1 dans le corps du patient, la face 3 de contact osseux du corps principal 2 du composant tibial 1 venant en regard de la zone osseuse plane préalablement préparée au niveau de l'extrémité distale du tibia Ti, puis on insère en force le moyen d'ancrage 4 du composant tibial 1 dans le pré-perçage tibial par impaction ;
- On positionne et maintient le composant talaire 6 dans le corps du patient, la face 30 talaire de contact osseux du composant talaire 6 venant en regard de la zone osseuse préalablement préparée au niveau du talus Ta, puis on insère en force le moyen d'ancrage 31 du composant talaire dans le ou les pré-perçages talaires par impaction ;
- On insère le composant intermédiaire 7 de la prothèse 5 dans le corps du patient, de sorte à l'intercaler entre le composant tibial 1 et le composant talaire 6, le tenon 19 du composant intermédiaire 7 coulissant dans la rainure 18 correspondante du composant tibial 1, de manière à solidariser le composant intermédiaire 7 au composant tibial 1 ;
- On referme le corps du patient par tout moyen adéquat et selon toute technique adaptée.

## Revendications

1. Composant (1) implantable de prothèse de cheville, ledit composant (1) comprenant un corps principal (2) pourvu d'une face (3) de contact osseux qui est prévue pour être agencée en contact avec une zone d'un corps osseux (Ti, Ta) d'une cheville et qui s'étend selon un plan (Pc) de contact médian, ledit composant (1) comprenant au moins un moyen d'ancrage (4) qui fait saillie de ladite face (3) de contact osseux pour ancrer ledit composant (1) dans ledit corps osseux (Ti, Ta), ledit moyen d'ancrage (4) comprenant deux paires (20A, 20B) d'ailes (21A, 22A, 21B, 22B) d'ancrage, qui s'étendent chacune respectivement dans un plan (P1, P2) entre une première extrémité (23A, 23B) reliée à ladite face (3) de contact osseux et une deuxième extrémité (24A, 24B) opposée, lesdits plans (P1, P2) d'extension respectifs des paires (20A, 20B) d'ailes (21A, 22A, 21B, 22B) d'ancrage étant sécants, lesdites deuxièmes extrémités (24A, 24B) des paires (20A, 20B) d'ailes (21A, 22A, 21B, 22B) d'ancrage étant inscrites dans un plan (Pa) d'attaque incliné par rapport audit plan (Pc) de contact médian, **caractérisé en ce que** les plans (P1, P2) dans lesquels lesdites paires (20A, 20B) d'ailes (21A, 22A, 21B, 22B) d'ancrage s'étendent sont orthogonaux audit plan (Pc) de contact médian de la face (3) de contact osseux.

2. Composant (1) selon la revendication précédente, ledit plan (Pa) d'attaque étant incliné par rapport au plan (Pc) de contact médian selon un angle (α) compris entre 3° et 30°, de préférence compris entre 3° et 15°, et de préférence encore compris entre 5° et 8°.

3. Composant (1) selon l'une quelconque des revendications précédentes, ladite face (3) de contact osseux s'étendant d'une part entre un bord antérieur (9) et un bord postérieur (10) opposé, et d'autre part entre un bord latéral (11) et un bord médial (12) opposé, ledit plan d'attaque (Pa) étant incliné par rapport audit plan (Pc) de contact médian selon une pente déclinante du bord antérieur (9) en direction du bord postérieur (10) de la face (3) de contact osseux.

4. Composant (1) selon l'une quelconque des revendications précédentes, les ailes (21A, 22A, 21B, 22B) de chacune desdites paires (20A, 20B) d'ailes (21A, 22A, 21B, 22B) d'ancrage étant conformées de manière que leurs projections orthogonales dans le plan (Pc) de contact médian sont symétriques entre elles par rapport au plan d'extension (P1, P2) de l'autre desdites paires (20A, 20B) d'ailes (21A, 22A, 21B, 22B).

5. Composant (1) selon l'une quelconque des revendications précédentes, les ailes (21A, 22A, 21B, 22B) de chacune des paires (20A, 20B) d'ailes du moyen d'ancrage (4) étant pourvues d'un bord extérieur latéral (25A, 26A, 25B, 26B) qui relie entre elles les première et deuxième extrémités (23A, 23B, 24A, 24B) des paires (20A, 20B) d'ailes, les bords extérieurs latéraux (25A, 26A, 25B, 26B) des ailes (21A, 22A, 21B, 22B) d'une même paire (20A, 20B) d'ailes (21A, 22A, 21B, 22B) s'étendant selon des directions (B-B', C-C', D-D', E-E') médianes obliques et convergentes en s'éloignant du plan (Pc) de contact médian.

6. Composant (1) selon les revendications 4 et 5, lesdites directions médianes d'extensions des bords extérieurs latéraux (25A, 26A, 25B, 26B) des ailes (21A, 22A, 21B, 22B) d'une même paire (20A, 20B) d'ailes (21A, 22A, 21B, 22B) étant sécantes selon un angle (β1, β2) compris entre 3° et 75°, de préférence compris entre 10° et 30°, et de préférence encore sensiblement égal à 20°.

7. Composant (1) selon l'une quelconque des revendications précédentes, les ailes (21A, 22A, 21B, 22B) du moyen d'ancrage (4) s'étendant entre lesdites première et deuxième extrémités (23A, 23B, 24A, 24B) selon une hauteur (Hmin, Hmax) au moins égale à 3 mm et au plus égale à 30 mm.

8. Composant (1) selon l'une quelconque des revendications précédentes, les ailes (21A, 22A, 21B, 22B) du moyen d'ancrage (4) présentant chacune une épaisseur (e1, e2) moyenne comprise entre 1 mm et 3 mm, de préférence égale à 1,5 mm environ.

9. Composant (1) selon l'une quelconque des revendications précédentes, les plans (P1, P2) d'extension desdites paires (20A, 20B) d'ailes (21A, 22A, 21B, 22B) étant perpendiculaires l'un à l'autre et sont, de préférence, respectivement agencés de manière sensiblement parallèle à des directions moyennes d'extension antéro-postérieure (AP) et latéro-médiale (LM) du corps principal (2) du composant (1) implantable, de sorte à définir respectivement une paire (20A) d'ailes (21A, 22A) antéro-postérieures et une paire (20B) d'ailes (21B, 22B) latéro-médiales.

10. Composant (1) selon l'une quelconque des revendications précédentes, ledit moyen d'ancrage (4) étant agencé sensiblement au centre de la face (3) de contact osseux du corps principal (2) du composant (1) implantable.

11. Composant (1) selon l'une quelconque des revendications précédentes, ledit moyen d'ancrage (4) formant une pièce monolithique avec le corps principal (2) dudit composant (1) implantable.

12. Composant (1) selon l'une quelconque des revendications précédentes, lesdites paires (20A, 20B) d'ailes (21A, 22A, 21B, 22B) étant reliées entre elles au niveau de l'intersection de leur plan (P1, P2) d'extension respectif par des congés (27A, 28A, 27B, 28B), lesquels présentent un rayon de courbure préférentiellement compris entre 2 mm et 15 mm.

13. Composant (1) selon l'une quelconque des revendications précédentes, le composant constituant un composant tibial (1), dont la face (3) de contact osseux est prévue pour être agencée en contact avec une zone d'une extrémité distale d'un tibia (Ti).

14. Prothèse (1) de cheville comprenant au moins un composant (1) implantable, ledit composant (1) implantable étant conforme à l'une quelconque des revendications 1 à 13.

15. Prothèse (1) de cheville selon la revendication précédente, ledit composant (1) implantable constituant un composant (1) tibial, dont la face (3) de contact osseux est prévue pour être agencée en contact avec une zone d'une extrémité distale d'un tibia (Ti), ladite prothèse (5) comprenant en outre un composant talaire (6) prévu pour être agencé en contact avec une zone d'un talus (Ta) correspondant.

## Patentansprüche

1. Implantierbare Komponente (1) für eine Knöchelprothese, wobei die genannte Komponente (1) einen Hauptkörper (2) umfasst, der mit einer Knochenkontaktfläche (3) versehen ist, die bereitgestellt ist, um in Kontakt mit einer Zone eines Knochenkörpers (Ti, Ta) eines Knöchels in Kontakt zu stehen und die sich in einer Kontaktmittenebene (Pc) erstreckt, wobei die genannte Komponente (1) mindestens ein Verankerungsmittel (4) umfasst, das von der genannten Knochenkontaktfläche (3) vorsteht, um die genannte Komponente (1) in dem genannten Knochenkörper (Ti, Ta) zu verankern, wobei das genannte Verankerungsmittel (4) zwei Paare (20A, 20B) von Verankerungsflügeln (21A, 22A, 21B, 22B) umfasst, die sich jeweils in einer Ebene (P1, P2) zwischen einem ersten Ende (23A, 23B), das mit der genannten Knochenkontaktfläche (3) verbunden ist, und einem gegenüberliegenden zweiten Ende (24A, 24B) erstrecken, wobei die genannten jeweiligen Ausdehnungsebenen (P1, P2) der Paare (20A, 20B) von Verankerungsflügeln (21A, 22A, 21B, 22B) einander schneiden, wobei die genannten zweiten Enden (24A, 24B) der Paare (20A, 20B) von Verankerungsflügeln (21A, 22A, 21B, 22B) in einer Angriffsebene (Pa) eingeschrieben sind, die in Bezug auf die genannte Kontaktmittenebene (Pc) geneigt ist, **dadurch gekennzeichnet, dass** die Ebenen (P1, P2), in denen sich die genannten Paare (20A, 20B) von Verankerungsflügeln (21A, 22A, 21B, 22B) erstrecken, orthogonal sind zu der genannten Kontaktmittenebene (Pc) der Knochenkontaktfläche (3).

2. Komponente (1) nach dem vorhergehenden Anspruch, wobei die genannte Angriffsebene (Pa) in Bezug auf die Kontaktmittenebene (Pc) unter einem Winkel (α) geneigt ist, der zwischen 3° und 30° beträgt, vorzugsweise zwischen 3° und 15° beträgt und bevorzugter zwischen 5° und 8° beträgt.

3. Komponente (1) nach einem der vorhergehenden Ansprüche, wobei sich die genannte Knochenkontaktfläche (3) einerseits zwischen einem anterioren Rand (9) und einem gegenüberliegenden posterioren Rand (10), und andererseits zwischen einem lateralen Rand (11) und einem gegenüberliegenden medialen Rand (12) erstreckt, wobei die genannte Angriffsebene (Pa) in Bezug auf die genannte Kontaktmittenebene (Pc) gemäß einer abnehmenden Steigung des anterioren Rands (9) in die Richtung des posterioren Rands (10) der Knochenkontaktfläche (3) geneigt ist.

4. Komponente (1) nach einem der vorhergehenden Ansprüche, wobei die Flügel (21A, 22A, 21B, 22B) von jedem der genannten Paare (20A, 20B) von Verankerungsflügeln (21A, 22A, 21B, 22B) derart ausgebildet sind, dass ihre orthogonalen Projektionen in der Kontaktmittenebene (Pc) zueinander symmetrisch in Bezug auf die Ausdehnungsebene (P1, P2) des anderen der genannten Paare (20A, 20B) von Flügeln (21A, 22A, 21B, 22B) sind.

5. Komponente (1) nach einem der vorhergehenden Ansprüche, wobei die Flügel (21A, 22A, 21B, 22B) von jedem der Paare (20A, 20B) von Flügeln des Verankerungsmittels (4) mit einem lateralen äußeren Rand (25A, 26A, 25B, 26B) versehen sind, der die ersten und zweiten Enden (23A, 23B, 24A, 24B) der Paare (20A, 20B) von Flügeln miteinander verbindet, wobei sich die lateralen äußeren Ränder (25A, 26A, 25B, 26B) der Flügel (21A, 22A, 21B, 22B) desselben Paars (20A, 20B) von Flügeln (21A, 22A, 21B, 22B) in die schrägen medianen und konvergenten Richtungen (B-B`, C-C', D-D', E-E') erstrecken, indem sie sich von der Kontaktmittenebene (Pc) entfernen.

6. Komponente (1) nach den Ansprüchen 4 und 5, wobei die genannten medianen Ausdehnungsrichtungen der lateralen äußeren Ränder (25A, 26A, 25B, 26B) der Flügel (21A, 22A, 21B, 22B) desselben Paars (20A, 20B) von Flügeln (21A, 22A, 21B, 22B) einander unter einem Winkel (β1, β2) schneiden, der zwischen 3° und 75° beträgt, vorzugsweise zwischen 10° und 30° beträgt und bevorzugter im Wesentlichen gleich 20° ist.

7. Komponente (1) nach einem der vorhergehenden Ansprüche, wobei sich die Flügel (21A, 22A, 21B, 22B) des Verankerungsmittels (4) zwischen den genannten ersten und zweiten Enden (23A, 23B, 24A, 24B) in einer Höhe (Hmin, Hmax) von mindestens gleich 3 mm und höchstens gleich 30 mm erstrecken.

8. Komponente (1) nach einem der vorhergehenden Ansprüche, wobei die Flügel (21A, 22A, 21B, 22B) des Verankerungsmittels (4) jeweils eine mittlere Dicke (e1, e2) aufweisen, die zwischen 1 mm und 3 mm beträgt, vorzugsweise ungefähr gleich 1,5 mm ist.

9. Komponente (1) nach einem der vorhergehenden Ansprüche, wobei die Ausdehnungsebenen (P1, P2) der genannten Paare (20A, 20B) von Flügeln (21A, 22A, 21B, 22B) zueinander rechtwinklig sind und vorzugsweise jeweils derart angeordnet sind, dass sie im Wesentlichen parallel zu den mittleren Ausdehnungsrichtungen antero-posterior (AP) und latero-medial (LM) des Hauptkörpers (2) der implantierbaren Komponente (1) sind, um jeweils ein Paar (20A) von antero-posterioren Flügeln (21A, 22A) und ein Paar (20B) von latero-medialen Flügeln (21B, 22B) zu definieren.

10. Komponente (1) nach einem der vorhergehenden Ansprüche, wobei das genannte Verankerungsmittel (4) im Wesentlichen im Zentrum der Knochenkontaktfläche (3) des Hauptkörpers (2) der implantierbaren Komponente (1) angeordnet ist.

11. Komponente (1) nach einem der vorhergehenden Ansprüche, wobei das genannte Verankerungsmittel (4) ein monolithisches Stück mit dem Hauptkörper (2) der genannten implantierbaren Komponente (1) bildet.

12. Komponente (1) nach einem der vorhergehenden Ansprüche, wobei die genannten Paare (20A, 20B) von Flügeln (21A, 22A, 21B, 22B) miteinander auf der Schnitthöhe ihrer jeweiligen Ausdehnungsebene (P1, P2) durch Auskehlungen (27A, 28A, 27B, 28B) verbunden sind, die einen Krümmungsradius aufweisen, der vorzugsweise zwischen 2 mm und 15 mm beträgt.

13. Komponente (1) nach einem der vorhergehenden Ansprüche, wobei die Komponente eine Tibiakomponente (1) bildet, deren Knochenkontaktfläche (3) bereitgestellt ist, um in Kontakt mit einer Zone eines distalen Endes einer Tibia (Ti) angeordnet zu sein.

14. Knöchelprothese (1), umfassend mindestens eine implantierbare Komponente (1), wobei die genannte implantierbare Komponente (1) gemäß einem der Ansprüche 1 bis 13 ausgebildet ist.

15. Knöchelprothese (1) nach dem vorhergehenden Anspruch, wobei die genannte implantierbare Komponente (1) eine Tibiakomponente (1) bildet, deren Knochenkontaktfläche (3) bereitgestellt ist, um in Kontakt mit einer Zone eines distalen Endes einer Tibia (Ti) angeordnet zu sein, wobei die genannte Prothese (5) außerdem eine Taluskomponente (6) umfasst, die bereitgestellt ist, um in Kontakt mit einer Zone eines entsprechenden Talus (Ta) angeordnet zu sein.

## Claims

1. An implantable ankle-prosthesis component (1), said component (1) comprising a main body (2) provided with a bone contact face (3) that is intended to be arranged in contact with an area of a bone body (Ti, Ta) of an ankle and that extends along a median contact plane (Pc), said component (1) comprising at least one anchoring means (4) that protrudes from said bone contact face (3) to anchor said component (1) into said bone body (Ti, Ta), said component (1) comprising two pairs (20A, 20B) of anchoring wings (21A, 22A, 21B, 22B), which each extend in a plane (P1, P2), respectively, between a first end (23A, 23B) connected to said bone contact face (3) and an opposite second end (24A, 24B), said respective planes of extension (P1, P2) of the pairs (20A, 20B) of anchoring wings (21A, 22A, 21B, 22B) intersecting each other, said second ends (24A, 24B) of the pairs (20A, 20B) of anchoring wings (21A, 22A, 21B, 22B) being inscribed in a leading plane (Pa) inclined with respect to said median contact plane (Pc), **characterized in that** the planes (P1, P2) in which said pairs (20A, 20B) of anchoring wings (21A, 22A, 21B, 22B) extend are orthogonal to said median contact plane (Pc) of the bone contact face (3).

2. The component (1) according to the preceding claim, wherein said leading plane (Pa) is inclined with respect to the median contact plane (Pc) by an angle (α) comprised between 3° and 30°, preferably comprised between 3° and 15°, and still preferably comprised between 5° and 8°.

3. The component (1) according to any one of the preceding claims, wherein said bone contact face (3) extends, on the one hand, between an anterior edge (9) and an opposite posterior edge (10), and on the other hand, between a lateral edge (11) and an opposite medial edge (12), said leading plane (Pa) being inclined with respect to said median contact plane (Pc) along a downslope from the anterior edge (9) to the posterior edge (10) of the bone contact face (3).

4. The component (1) according to any one of the preceding claims, wherein the wings (21A, 22A, 21B, 22B) of each of said pairs (20A, 20B) of anchoring wings (21A, 22A, 21B, 22B) are shaped so that their orthogonal projections on the median contact plane (Pc) are symmetrical to each other with respect to the plane of extension (P1, P2) of the other of said pairs (20A, 20B) of anchoring wings (21A, 22A, 21B, 22B).

5. The component (1) according to any one of the preceding claims, wherein the wings (21A, 22A, 21B, 22B) of each of said pairs (20A, 20B) of wings of the anchoring means (4) are provided with a lateral external edge (25A, 26A, 25B, 26B) that connects to each other the first and second ends (23A, 23B, 24A, 24B) of the pairs (20A, 20B) of wings, the lateral external edges (25A, 26A, 25B, 26B) of the wings (21A, 22A, 21B, 22B) of a same pair (20A, 20B) of wings (21A, 22A, 21B, 22B) extending along median directions (B-B', C-C', D-D', E-E') that are oblique and convergent as they go away from the median contact plane (Pc).

6. The component (1) according to claims 4 and 5, wherein said median directions of extensions of the lateral external edges (25A, 26A, 25B, 26B) of the wings (21A, 22A, 21B, 22B) of a same pair (20A, 20B) of wings (21A, 22A, 21B, 22B) intersect each other by an angle (β1, β2) comprised between 3° and 75°, preferably comprised between 10° and 30°, and still preferably substantially equal to 20°.

7. The component (1) according to any one of the preceding claims, wherein the wings (21A, 22A, 21B, 22B) of the anchoring means (4) extend between said first and second ends (23A, 23B, 24A, 24B) over a height (Hmin, Hmax) at least equal to 3 mm and at most equal to 30 mm.

8. The component (1) according to any one of the preceding claims, wherein the wings (21A, 22A, 21B, 22B) of the anchoring means (4) each have a mean thickness (e1, e2) comprised between 1 mm and 3 mm, preferably equal to about 1.5 mm.

9. The component (1) according to any one of the preceding claims, wherein the planes of extension (P1, P2) of said pairs (20A, 20B) of wings (21A, 22A, 21B, 22B) are perpendicular to each other and are, preferably, respectively arranged substantially parallel to mean anteroposterior (AP) and lateromedial (LM) directions of extension of the main body (2) of the implantable component (1), so as to define respectively a pair (20A) of anteroposterior wings (21A, 22A) and a pair (20B) of lateromedial wings (21B, 22B).

10. The component (1) according to any one of the preceding claims, wherein said anchoring means (4) is arranged substantially at the centre of the bone contact face (3) of the main body (2) of the implantable component (1).

11. The component (1) according to any one of the preceding claims, wherein said anchoring means (4) forms a monolithic part with the main body (2) of said implantable component (1).

12. The component (1) according to any one of the preceding claims, wherein said pairs (20A, 20B) of wings (21A, 22A, 21B, 22B) are connected to each other at the intersection of their respective planes of extension (P1, P2) by fillets (27A, 28A, 27B, 28B), which have a radius of curvature preferentially comprised between 2 mm and 15 mm.

13. The component (1) according to any one of the preceding claims, wherein the component constitutes a tibial component (1), whose bone contact face (3) is intended to be arranged in contact with an area of a distal end of a tibia (Ti).

14. An ankle prosthesis (5) comprising at least one implantable component (1), wherein said implantable component (1) is in accordance with any one of claims 1 to 13.

15. The ankle prosthesis (5) according to the preceding claim, wherein said implantable component (1) constitutes a tibial component (1), whose bone contact face (3) is intended to be arranged in contact with an area of a distal end of a tibia (Ti), said prosthesis (5) further comprising a talar component (6) intended to be arranged in contact with an area of a corresponding talus (Ta).
